Europäisches Patentamt

European Patent Office

Office européen des brevets

(19)

(11) Veröffentlichungsnummer: **0 258 684 B1**

(12) **EUROPÄISCHE PATENTSCHRIFT**

(45) Veröffentlichungstag der Patentschrift: 30.10.91

(51) Int. Cl.⁵: **D04H 1/00**, D04H 1/60

(21) Anmeldenummer: 87111503.6

(22) Anmeldetag: 08.08.87

(54) Verfahren zur Herstellung eines Wirrfaserstoffs aus Glasfasern und Polymer für die Herstellung glasfaserverstärkter Kunststofformteile sowie Vorrichtung zur Durchführung des Verfahrens.

(30) Priorität: 01.09.86 DE 3629748
10.02.87 DE 3704035

(43) Veröffentlichungstag der Anmeldung:
09.03.88 Patentblatt 88/10

(45) Bekanntmachung des Hinweises auf die
Patenterteilung:
30.10.91 Patentblatt 91/44

(84) Benannte Vertragsstaaten:
AT BE CH DE ES FR GB IT LI LU NL SE

(56) Entgegenhaltungen:
EP-A- 0 007 149
FR-A- 2 316 364
FR-E- 64 647
US-A- 2 827 668

(73) Patentinhaber: Menzolit GmbH
Bahnhofstrasse 31
W-7527 Kraichtal-Menzingen(DE)

(72) Erfinder: Ehnert, Gerd
Brucknerstrasse 6
W-7523 Graben-Neudorf(DE)
Erfinder: Ehlers, Manfred, Dipl.-Ing.
Friedrichstrasse 77
W-7527 Kraichtal-Unteröwisheim(DE)
Erfinder: Sauer, Gerhard
Ahornweg 8
W-7520 Bruchsal(DE)
Erfinder: Vogel, Klaus
Burgstrasse 7
W-7527 Kraichtal-Menzingen(DE)

(74) Vertreter: Dr.-Ing. Hans Lichti Dipl.-Ing. Heiner
Lichti Dipl.-Phys. Dr. Jost Lempert
Postfach 41 07 60 Durlacher Strasse 31
W-7500 Karlsruhe 41(DE)

## Beschreibung

Die Erfindung betrifft ein Verfahren zur Herstellung eines Wirrfaserstoffs aus geschnittenen Faserbündeln und einem Bindemittel auf Polymerbasis als Ausgangsprodukt für die Herstellung faserverstärkter Kunststofformteile, wobei die Faserbündel auffilamentiert und verwirbelt und dabei mit dem Bindemittel versehen werden sowie eine Vorrichtung zur Durchführung des Verfahrens.

Die Erfindung betrifft zwar in erster Linie die Verarbeitung von geschnittenen Glasfasern, ist aber in gleicher Weise auf andere anorganische Fasern oder organische Fasern, wie Kohlefasern bei der Erfindungsdarstellung, Aramidfasern oder auch Polyesterfasern anwendbar. Soweit von Glasfasern gesprochen wird, können diese auch durch die vorgenannten, gleichwirkenden Fasern ersetzt werden.

Geschnittene Glasfasern werden in großem Umfang zu glasfaserverstärkten Kunststofformteilen verarbeitet. Hierbei ergeben sich Probleme bei der Aufbereitung der Glasfasern zu einem Wirrfaserstoff, da die Glasfaser zumindestens gegenüber organischen Fasern vergleichsweise biegesteif ist. Hinzu kommt, daß die Glasfasern von multifilen Fasersträngen auf Länge geschnitten werden, so daß hierbei Glasfaserbündel entstehen, innerhalb der die einzelnen Glasfasern oder Einzelfasern parallel und eng aneinander liegen. Diese Glasfaserbündel lassen sich nur mit Schwierigkeiten zu wirr liegenden Einzelfasern aufbereiten. In der Praxis geschieht die Wirrfaserstoffbildung entweder durch Ausstreuen der Fasern zu einem Vlies und einem Imprägnieren des Vlieses mit flüssigem Kunstharz oder es werden die Fasern in flüssiger Phase zu einer Aufschlämmung verarbeitet, die durch Rühr- oder Mischbewegung zu einem Wirrfaserstoff verarbeitet wird. Auch hierbei werden entweder flüssige Kunstharze oder aber pulvrige Bindemittel in Verbindung mit einer wssrigen Aufschlämmung eingesetzt. Im letztgenannten Fall wird die Aufschlmmung nach Bildung eines Wirrfaservlieses getrocknet.

Es ist ferner vorgeschlagen worden (P 36 04 888.7), flüssige Benetzungsmittel mit einem Anteil von maximal 20 Gew.-% zuzusetzen, um so eine feuchte, jedoch noch rieselfähige Masse zu erhalten, die als Zwischenprodukt in Kunststoffbeuteln verpackt an den Weiterverarbeiter weitergeben oder in einer beheizten Bandpresse zu vorgeformten Plattenmaterial verarbeitet werden kann. Aus diesen Zwischenprodukten lassen sich dann die gewünschten glasfaserverstärkten Kunststofformteile herstellen.

Die EP-A-7 149 zeigt ein Verfahren zum Imprägnieren von Fasern mit trockenem puderförmigen Harz. Hierbei wird ein von einer Endlosspule abgewickelter Faden zerschnitten und über eine Venturidüse einer Mischkammer zugeführt In der Mischkammer wird von der Seite her durch einen Aufgabetrichter das Harz beigefügt. Durch eine weitere Venturidüse gelangt die Mischung in einen Ablagebehälter, in dem die Geschwindigkeit der Faser-Harz-Mischung abgebaut wird, so daß die beschichteten Fasern sich im Behälter ablegen. Die Förderung der Fasern geschieht dadurch, daß im Bereich der engsten Stellen der Venturidüsen diesen Druckluft zugeführt wird.

Aufgrund des schnellen Durchströmens der Faser durch die Mischkammer und des nachträglichen Eintrags des Harzes kann lediglich eine sehr geringe Beschichtung der Fasern mit Harz erreicht werden. Darüber hinaus ergibt sich kein homogener Wirrfaserstoff mit gleich verteilten Faseranteilen und auf diesen anhaftenden Harzanteilen. Vielmehr ergeben sich zwei Fasern, nämlich einerseits mit wenig Harz beschichtete Fasern, andererseits Harzcluster, die sich in der Kammer oder bei der Weiterverarbeitung sehr schnell entmischen werden.

Der Erfindung liegt die Aufgabe zugrunde, ein gattungsgemäßes Verfahren zu schaffen, bei dem zuverlässig die Fasern sowohl hinreichend dicht mit Bindemitteln versehen werden als auch separate Haufen oder Cluster von Bindemitteln und damit eine Entmischung verhindert wird.

Erfindungsgemäß wird die genannte Aufgabe bei einem Verfahren der eingangs genannten Art dadurch gelöst, daß jeweils eine Charge aus aufeinander abgestimmten Mengen von Fasern und Bindemitteln diskontinuierlich in eine Wirbelkammer eingegeben, die Charge dort durch einen zugeführten turbulenten Luftstrom verwirbelt und anschließend als solche zur Weiterverarbeitung ausgegeben wird. Eine Vorrichtung zur Durchführung des Verfahrens ist dadurch gekennzeichnet, daß die Wirbelkammer luftdurchlässige Wände aus Filtermaterial und eine mit einem Schieber versehene Austrittsöffnung für den Wirrfaserstoff aus Faser und Bindemittel aufweist.

Praktische Versuche haben gezeigt, daß die sonst stark richtungsorientierten und aneinander haftenden Glasfaserbündel mit dem erfindungsgemäßen Druckluftverfahren zu einem Wirrfaserstoff aufgeschlossen, also auffilamentiert werden können, bei dem einerseits die kurzen Einzelfasern vollständig vereinzelt sind, andererseits eine völlig irreguläre Anordnung und Verteilung einnehmen. Aus den geschnittenen Glasfaserbündeln läßt sich auf diese Weise Weise ein voluminöser watteähnlicher Faserstoff herstellen, der sich durch ein Höchstmaß an Desorientierung der einzelnen Glasfasern auszeichnet.

Anläßlich der vorgenannten Herstellung des Wirrfaserstoffs wird später zugleich mit dem Verwirbeln auch das Kunststoffbindemittel in Pulverform zugegeben wird. Es hat stoffbindemittel in Pulverform zugegeben, da beim Aufschließen der Glasfasern durch Verwirbeln im Druckluftstrom bei gleichzeitigem Eintrag des Bindemittelpulvers eine hervorragend homogene Mischung entsteht, in der das Bindemittelkorn an den Glasfasern haftet bzw. in dem watteförmigen Wirrfaserstoff in homogener Verteilung festgehalten wird. Mit Vorteil wird das Bindemittel zusammen mit den Glasfasern in die Luftströmung eingegeben. Bei der Verwirbelung werden die Glasfasern und das Bindemittel aufgrund ihrer unterschiedlichen Struktur (Fasern bzw. Pulverkörnchen) unterschiedlich beschleunigt und so die Bindemittelteilchen praktisch in die Glasfasern hineingeschossen. Eine vollständige homogene Durchmischung wird nicht erreicht, wenn zunächst ein Verwirbeln der Fasern erfolgt und erst anschließend das Bindemittel zugegeben und eine Vermischung versucht wird. Eine solche wird dann bestenfalls lokal erreicht.

Durch das erfindungsgemäße Vorgehen wird zunächst eine mechanische Verankerung des Bindemittels zwischen den Glasfasern bewirkt, wodurch die Bindemittelkörner oder -pulverteilchen von Glasfasern umgeben und zwischen diesen gehalten werden. Die mechanische Verankerung kann weiterhin dadurch verbessert werden, daß pulverförmiges Bindemittel mit einer kantigen und rauhen Oberflächengestalt zugeführt wird. In weiterer bevorzugter Ausgestaltung ist vorgesehen, daß beim Verwirbeln die Komponenten elektrostatisch aufgeladen werden. Hierdurch wird ein elektrostatisches Aneinanderhaften der Komponenten erreicht.

Vorteilhafterweise liegt die Rezeptur des Bindemittelpulvers in den folgenden Bereichen:

Thermoplast-Pulver 50 bis 90 Gew.-%, Ruß 0 bis 15 Gew.-%, Antioxidantien 0 bis 5 Gew.-% und sonstige, wie mineralische Füllstoffe, insbesondere Kreide, Talkum oder dergleichen 0 bis 30 Gew.-%.

Der auf erfindungsgemäße Weise erhaltene Wirrfaserstoff, der, wie gesagt, von watteförmiger Konsistenz ist, wird vorzugsweise zu einem filzartigen Gebilde verdichtet. In dieser Form ist dann der Wirrfaserstoff für die Weiterverarbeitung handhabbar, beispielsweise kann er in Folienpackungen abgepackt und an den Verarbeiter weitergegeben oder aber unmittelbar nach der Vliesherstellung unter Anwendung von Druck und Wärme zu einem Plattenmaterial als Zwischenprodukt weiterverarbeitet werden. Die Verdichtung kann insbesondere dadurch erfolgen, daß die Abpackung des Wirrfaserstoffs unter Vakuum in Beuteln erfolgt. Soweit das Bindemittel mit den Fasern verwirbelt wurde wird hierdurch eine Phasentrennung von Fasern und Bindemitteln während Lagerung und Transport weitgehend ausgeschlossen.

Bei der erfindungsgemäßen Vorrichtung werden die Glasfasern über die Aufgabeeinrichtung gemäß einer Ausgestaltung in die Druckluftleitung eingegeben und von dem Druckluftstrom mitgerissen. Dieser Druckluftstrom wird dann in der anschließenden Wirbelkammer in eine starke turbulente Wirbelströmung versetzt, wobei die Glasfasern vereinzelt und in eine lockere Wirrlage gebracht werden. Alternativ können die Glasfasern direkt in die Wirbelkammer eingegeben werden.

Mit Vorzug zeichnet sich die vorgenannte Vorrichtung aus durch wenigstens eine Aufgabeeinrichtung für das Bindemittelpulver, so daß innerhalb der Wirbelkammer in einem Arbeitsgang nicht nur das Aufschließen der Glasfasern zu einem Wirrfaserstoff, sondern zugleich auch das Vermischen mit dem trokkenen Bindemittel erfolgt.

In weiterer vorteilhafter Ausgestaltung der erfindungsgemäßen Vorrichtung weist die Druckluftleitung ein in die Wirbelkammer reichendes Mündungsstück auf, das in ändernder Richtung mit Bezug auf die Wirbelkammer beweglich ist. Das Mundstück kann beispielsweise quer zur Strömungsachse in eine oszillierende Bewegung oder auch in eine um die Strömungsachse rotierende Bewegung versetzt werden, wodurch innerhalb der Wirbelkammer eine sich ständig ändernde Wirbelströmung entsteht. Hierdurch ist eine besonders effektive und schnelle Herstellung des Wirrfaserstoffs möglich.

In weiterer Ausgestaltung der Erfindung ist vorgesehen, daß die Wirbelkammer als Behälter mit einer sich über den Behälterquerschnitt erstreckenden Austragsöffnung ausgebildet ist und insbesondere daß ein in die Wirbelkammer von der der Austragsöffnung gegenüberliegenden Seite eintauchender und bis zur Austragsöffnung beweglicher Austragsschieber vorgesehen ist. Mittels dieses Austragsschiebers läßt sich der sich im Behälter ansammelnde Wirrfaserstoff in einfacher Weise austragen.

Gemäß einem weiteren Merkmal der erfindungsgemäßen Vorrichtung ist an die Austragsöffnung eine Verdichtungseinrichtung angeschlossen, um aus dem Wirrfaserstoff den vorverdichteten Filz herzustellen.

Diese Verdichtungseinrichtung kann mit Vorteil von dem Austragsschieber und einer mit Abstand von der Austragsöffnung angeordneten Gegendruckplatte gebildet sein. Auf diese Weise wird der Wirrfaserstoff unmittelbar nach dem Austragen aus der Wirbelkammer zu einem Filz vorgeformt.

An die Verdichtungseinrichtung kann entweder eine beheizte Bandpresse zur Weiterverarbeitung des verdichteten Glasfaserfilzes zu glasfaserverstärkten Kunststoffplatten als Zwischenprodukt für die Herstellung von Formteilen oder aber eine Einrichtung zum Abpacken des Glasfaserfilzes in Folienpackungen angeschlossen sein. Im ersten Fall entstehen als Zwischenprodukt handhabungsfähige glasfaserverstärkte Kunststoffplatten, die unmittelbar nach ihrer Herstellung in eine beheizte Formpresse eingegeben oder aber an einen Weiterverarbeiter geliefert werden können. Im zweiten Fall kann der Filz in der ihm verliehenen Form verpackt werden. Die Verpackungsfolie besteht vorzugsweise aus dem gleichen Material wie die Matrix (Thermoplast) des Filzes oder aus einem mit diesem kompatiblen, verträglichen Material, so daß sie bei der Formteilherstellung gleich mitverarbeitet werden kann.

Bei der Weiterverarbeitung des erfindungsgemäß hergestellten Wirrfaserstoffs zu glasfaserverstärkten Kunststofformteilen hat sich gezeigt, daß gegenüber den in herkömmlicher Weise hergestellten Produkten sowohl eine Erhöhung des Biege-E-Moduls als auch eine Erhöhung der Biegefestigkeit erreicht wird. Die Ursache hierfür dürfte in dem wesentlich besseren Aufschluß der Glasfaserbündel und der homogeneren Mischung mit dem trockenen Bindemittel zu sehen sein. Insbesondere können auch höherwertige, sogenannte technische Thermoplaste mit einer besseren Wärmestandfestigkeit als das bisher regelmäßig eingesetzte Polypropylen verarbeitet werden. Insbesondere können beim erfindungsgemäßen Verfahren die einzelnen Komponenten schnell und in kleinen Chargen gewechselt oder in ihrer Menge verändert werden, so daß Matrix (Thermoplast) und Verstärkungstyp jederzeit an das jeweilige Anforderungsprofil der Formteile - beispielsweise beim Auswechseln der Werkzeuge zur Herstellung der Formteile - optimal angepaßt werden können. Das erfindungsgemäße Verfahren bietet die Möglichkeit zur Herstellung von glasfaserverstärkten Produkten bei reduzierten Investitionskosten und Energieaufwand, insbesondere da einer Verwirbelungskammer auch mehrere unterschiedliche Formwerkzeuge nachgeordnet werden können.

Ein wesentlicher Vorteil des durch das erfindungsgemäße Verfahren erzeugte filz- oder watteartigen Zwischenprodukts besteht darin, daß durch die Wirrlage der Einzelfasern und nicht nur von Bündeln derselben, wie beim Stande der Technik eine bessere Fließfähigkeit des erhitzten Produkts bei der Weiterverarbeitung gegeben ist.

Das erfindungsgemäße Verfahren schafft ein Erzeugnis zur Herstellung faserverstärkter Thermoplastteile in Form eines watteartigen Filz, in dem die Einzelfasern nahezu vollständig vereinzelt sind und eine irreguläre Anordnung und Verteilung einnehmen. Das gleiche gilt für ein durch die erwähnte Weiterverarbeitung erzeugtes Halbzeug zur Herstellung faserverstärkter Thermoplastteile sowie ein solches faserverstärktes Thermoplastformteil selbst.

Weitere Halbzeugausgestaltungen und Formteile können dabei im Anschluß an die erfindungsgemäßen Verfahrensschritte grundsätzlich weiter in bekannter Weise hergestellt sein.

Nachstehend ist die Erfindung anhand von in der Zeichnung wiedergegebenen Ausführungsbeispielen beschrieben. In der Zeichnung zeigen:

Fig. 1    ein Schema eines bevorzugten Verfahrensablaufs;

Fig. 2    eine schematische Ansicht der Vorrichtung einer ersten Ausführungsform;

Fig. 3    eine schematische Ansicht einer zweiten Ausführungsform der Vorrichtung;

Fig. 4    eine schematische Darstellung einer weiteren Ausführungsform der erfindungsgemäßen Vorrichtung; und

Fig. 5    eine gegenüber der Ausgestaltung der Figur 2 abgewandelte Verpackungseinrichtung zur Verpackung des Wirrfaserstoffes.

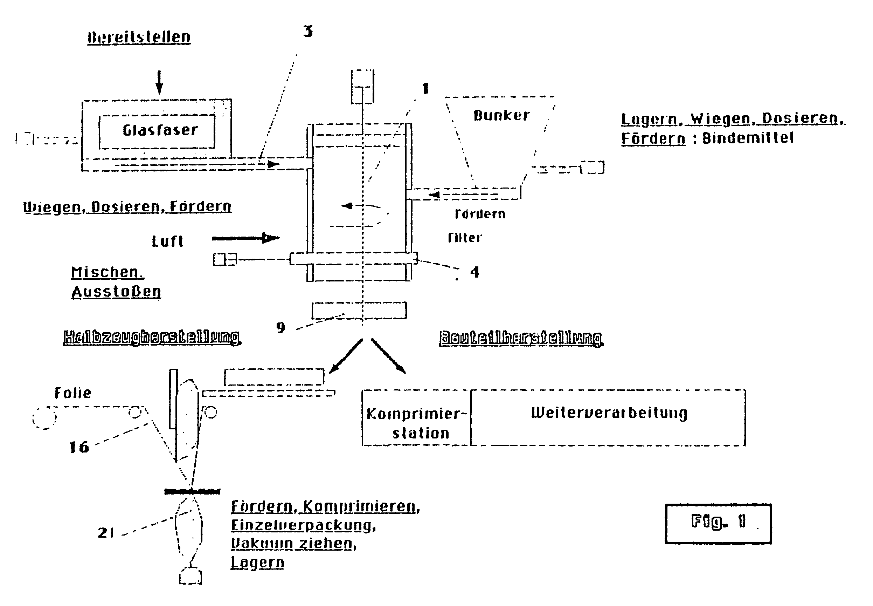

Die Figur 1 zeigt ein Ablaufschema einer bevorzugten Ausgestaltung des erfindungsgemäßen Verfahrens. Ausgegangen wird einerseits von Glasfasern, die bereitgestellt und über eine Aufgabeeinrichtung 3 zugeführt werden. Statt Glasfasern können auch Kohlefasern, Aramidfasern, Polyesterfasern oder dergleichen verwendet werden. Weiterhin wird das Bindemittel bereitgestellt. Dieses weist zunächst einen Thermoplasten, wie Polypropylen als Matrix auf. Hier ist lediglich zu beachten, daß der Thermoplast als Pulver und bestenfalls grießförmig zugeführt werden muß, so daß gegebenenfalls ein Granulat weiter mittels einer Mühle zu verfeinern ist. Weiterhin kann das Bindemittel Ruß, Wachs sowie sonstige Additive enthalten. Die einzelnen Komponenten werden in herkömmlicher Weise in einem HeizKühlmischer miteinander vermischt und in einem Bunker bereitgestellt. Die einzelnen Komponenten, nämlich Glasfaser und Bindemittel werden dosiert, der Wirbelkammer 1 zugeführt, in die ebenfalls Druckluft zur Verwirbelung eingeblasen wird. Da in der Wirbelkammer 1 kein erhöhter Druck aufgebaut, sondern lediglich die einzelnen Komponenten verwirbelt werden sollen, bestehen seine Wände aus Filtermaterial, durch welches die eingeblasene Luft austreten kann, welches aber die Materialkomponenten zurückhält.

Nach der Darstellung werden Glasfasern, Bindemittel und Luft jeweils separat der Wirbelkammer 1 zugeführt, stattdessen können Glasfasern und Bindemittel auch gemeinsam in einen Aufgabetrichter hineindosiert und gemeinsam der Wirbelkammer zugeführt werden. Sie können auch nicht der Wirbelkammer direkt, sondern stattdessen in eine Druckluftleitung, die zur Wirbelkammer 1 führt, eingespeist werden, dies wird weiter unten noch im einzelnen erläutert.

Nach hinreichender Verwirbelung der Komponenten in der Wirbelkammer 1 wird eine Austragsöffnung (mittels des Schiebers 4) geöffnet und das verwirbelte Material aus der Wirbelkammer 1 als "Wattebausch" oder Filz 9 ausgestoßen.

Zur Halbzeugherstellung wird der Wattebausch oder Filz 9 zwischen Folien 16 geführt und durch diese unter Vakuum in endlos aneinander gereihten Beuteln 21 verpackt.

Derart verpackt sind die dosierten Filze 9 lager- und transportfähig, ohne daß eine nachteilige Trennung der einzelnen Komponenten zu befürchten ist.

Alternativ kann sogleich eine Fertigwaren- bzw. Bauteilherstellung erfolgen. Hierzu wird der Filz 9 einer Komprimierstation zugeführt und anschließend in geeigneter Weise weiterverarbeitet, nämlich geschmolzen, gegebenenfalls dosiert, einer Presse zugeführt und in dieser in ansich bekannter Weise zum Formteil gepreßt.

Während die Parameter der zugeführten Glasfaserbündel in einen größeren Bereich je nach gewüschten Eigenschaften des Fertigprodukts, wie Elastizitätsmodul und Biegefestigkeit, variiert werden können, werden bevorzugter Weise Faserbündel mit einer Länge von 4 bis 25 mm und einer Texturierung von 5,5 bis 300 Tex. eingesetzt. Die Faserbündel können dabei aus 200 bis 800 Einzelfilamenten mit Einzelfadendurchmessern in der Größenordnung von 5 bis 20, vorzugsweise 8 bis 14 um liegen.

Die gute Verwirbelung und damit die Möglichkeit zur Wirrlagenbildung zeigt sich insbesondere daraus, daß bei Zuführung von Glasfasern im vorstehend genannten Bereich mit einer Schüttdichte von 600 bis 800g pro Liter und Bindemittel mit einer Schüttdichte von 500g pro Liter das nach der Verwirbelung entstehende "Watte-Produkt" vor Komprimierung eine Dichte von 20g pro Liter aufweist. Die in die Wirbelkammr eingeblasene Luft kann mit einem Druck, der in weiten Grenzen variierbar ist, zugeführt werden, wobei der Druck allerdings nicht unter 0,5 bar liegen sollte, da dann keine hinreichende Verwirbelung mehr erreichbar ist. Einerseits wird mit höherem Druck die Vermischung verbessert und andererseits eine gleichgute Mischung bei höherem Druck in kürzerer Zeit erreicht. Bevorzugter Weise wird daher ein Druck in der Größenordnung von 7 bis 10 bar eingesetzt, wobei eine gute Vermischung mit der vorstehend genannten Schüttdichte bei den ebenfalls erwähnten Ausgangskomponenten bei 7 bar in einer Zeit von 10 bis 15 Sekunden erreichbar ist.

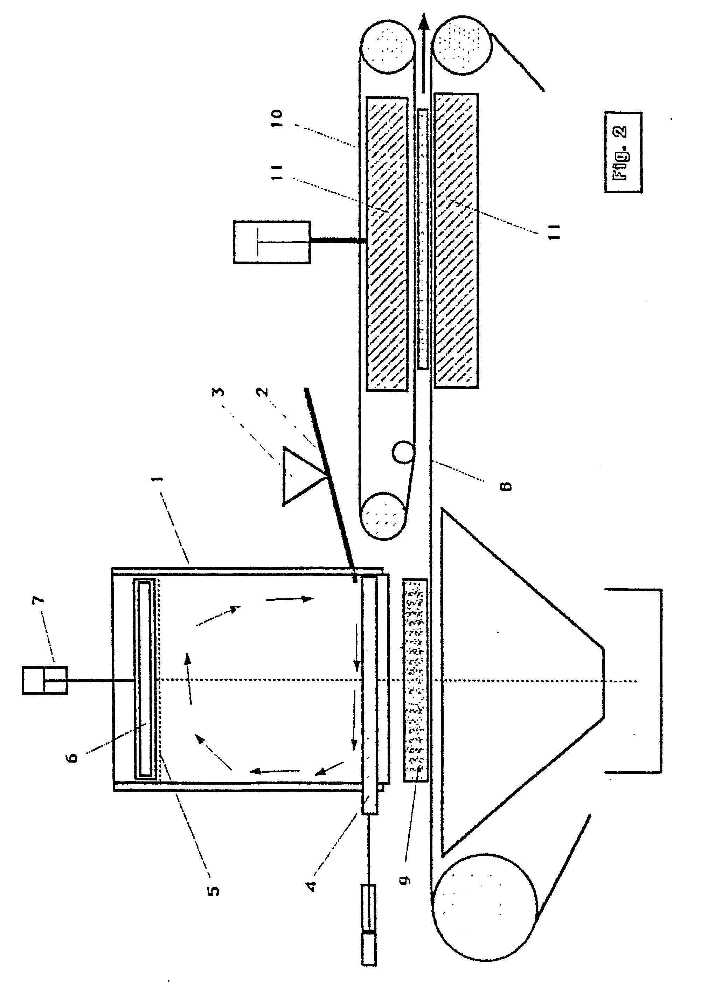

Die in Figur 2 gezeigte Vorrichtung weist als Kernstück eine behälterartige Wirbelkammer 1 auf, in die eine Druckluftleitung 2 nahe dem Boden einmündet. Die Druckluftleitung 2 ist mit einer Aufgabeeinrichtung, z.B. in Form eines Aufgabetrichters 3 versehen, in die eine auf die Größe der Wirbelkammer abgestimmte Menge an Glasfasern und eine entsprechend dosierte Menge an Bindemittelpulver eingegeben wird. Glasfasern und Bindemittel werden beispielsweise durch Injektorwirkung von dem Druckluftstrom in der Leitung 2 mitgerissen. Der Boden der Wirbelkammer 1 ist von einem Schieber 4 gebildet, der in der gezeigten Lage den Behälter nach unten abschließt und in der nicht gezeigten geöffneten Lage den gesamten Behälterquerschnitt freigibt. An seiner Oberseite ist der Behälter 1 mit einer Entlüftung versehen, die beispielsweise durch ein Filter 5 gebildet ist. Hinter dem Filter sitzt ein Austragschieber 6, der den Querschnitt des Behälters 1 etwa ausfüllt und mittels eines Hubzylinders 7 angetrieben ist.

Die in die Aufgabeeinrichtung 3 zugegebenen Glasfasern und das Bindemittelpulver strömen mit hoher Geschwindigkeit in den Behälter 1 ein und werden dort in eine irreguläre Wirbelströmung, wie sie mit Pfeilen angedeutet ist, umgelenkt. Es bildet sich mit der Zeit innerhalb des Behälters 1 ein Wirrfaserstoff aus den aufgeschlossenen Glasfaserbündeln, der zugleich mit dem Bindemittelpulver homogen gemischt ist.

Bei dem Ausführungsbeispiel gemäß Figur 2 läuft unterhalb des Behälters 1 ein Förderband 8 um, wobei unmittelbar unterhalb des Behälters 1 und unter dem Förderband eine nicht gezeigte Gegendruckplatte angeordnet ist. Diese bildet zusammen mit dem Austragschieber 6 eine Verdichtungseinrichtung. Nach Öffnen des Schiebers 4 und Freigabe der Austragsöffnung fährt der Austragsschieber 6 nach unten und schiebt den voluminösen Wirrfaserstoff vor sich her und verdichtet ihn gegen die unterhalb des Förderbandes 8 befindliche Gegendruckplatte zu einem Filz 9. Der Filz 9 wird im Taktbetrieb einer Bandpresse 10 zugeführt, die mit oberen und unteren Heizeinrichtungen 11 versehen ist, so daß der Filz 9 zu einer glasfaserverstärkten Kunststoffplatte als Zwischenprodukt verdichtet wird.

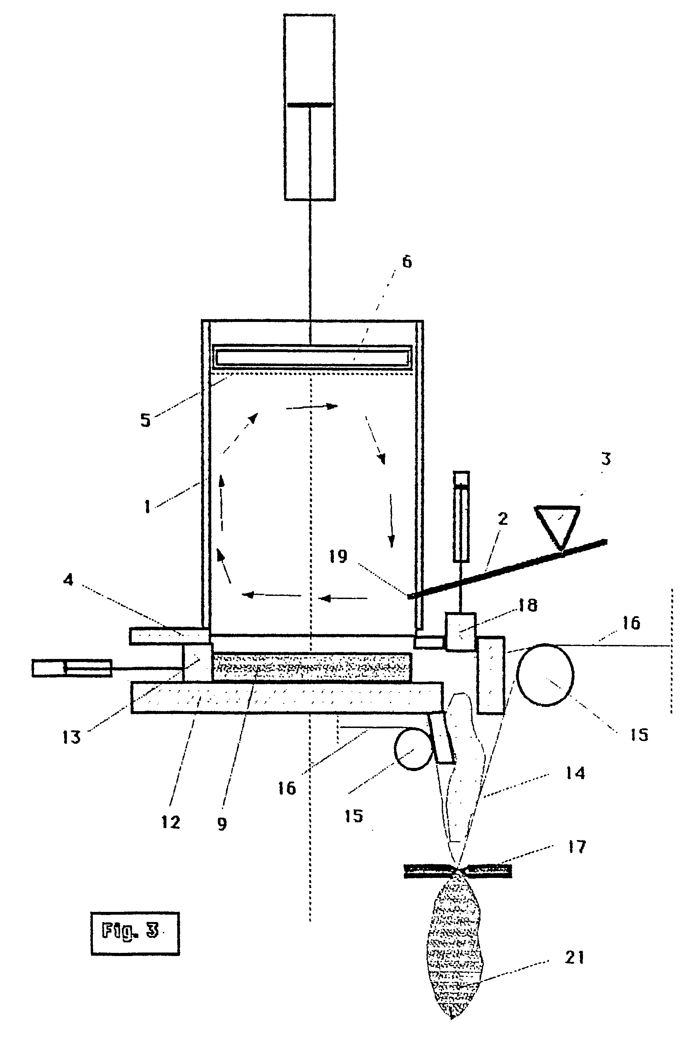

Bei der Ausführungsform gemäß Figur 3 ist die ein Teil der Verdichtungseinrichtung bildende Gegendruckplatte 12 erkennbar. Ansonsten entsprechen die Wirbelkammer und die ihr zugeordneten Bauteile im wesentlichen der Ausrührungsform gemäß Figur 1. In Abwandlung gegenüber dieser Vorrichtung läuft über der Gegendruckplatte 12 ein Transportschieber 13, der den vorverdichteten Filz von der Gegendruckplatte 12 herunterfördert. Hinter der Gegendruckplatte 12 ist eine Verpackungseinrichtung 14 angeordnet, die zwei Zuführrollen 15 für je eine Folie 16 sowie eine Schweißeinrichtung 17 und einen Packzylinder 18 aufweist. Der mittels des Schiebers 13 von der Gegendruckplatte 12 abgeschobene Filz 9 wird von den Packzylindern 18 zwischen die beiden Folien 16 eingestampft und anschließend die Folie taktweise weitertransportiert, wobei die Schweißeinrichtung 17 die entstehenden Wirrfaserstoff-Portionen zwischen die Folien einschließt.

Um die Verwirbelung der Glasfasern innerhalb des Behälters 1 besonders effektiv zu gestalten, kann die Druckluftleitung 2 ein in den Behälter 1 hineinragendes Mundstück 19 aufweisen, dessen Richtung durch Bewegen des Mundstücks veränderbar ist, so daß vermieden wird, daß sich ein gleich bleibender Wirbel innerhalb des Behälters 1 bildet.

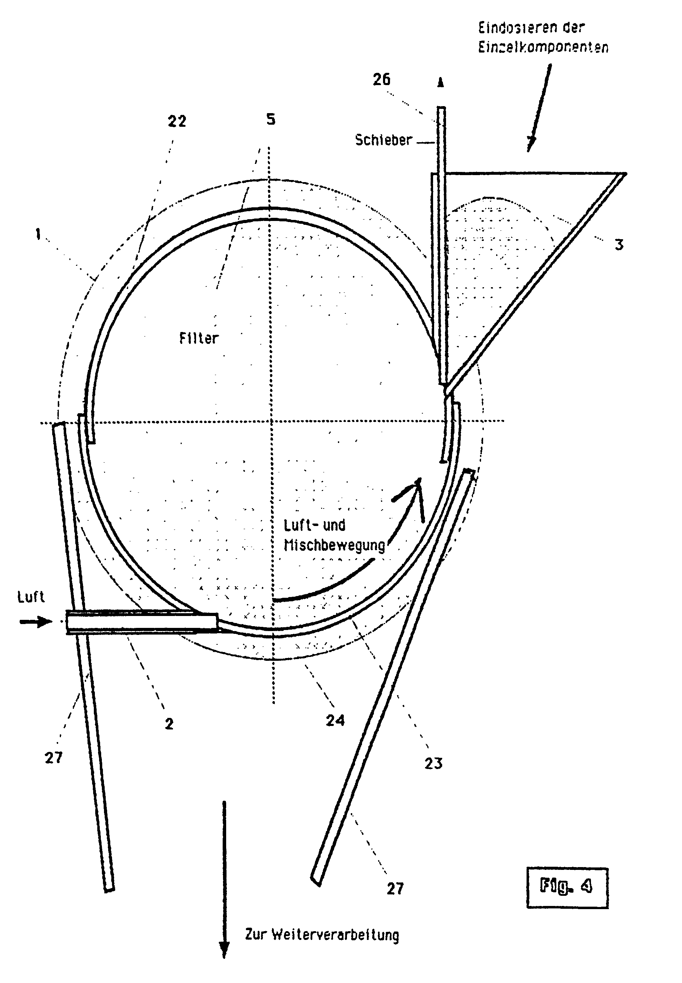

Die Figur 4 zeigt eine alternative Ausgestaltung der Wirbelkammer 1. Die Wirbelkammer 1 ist als liegender Zylinder ausgebildet, der an seinen Stirnseiten mit dem Filter 5 bespannt ist. Die obere Zylindermantelhälfte 22 ist fest und undurchlässig. Die untere Zylinderhälfte 23 ist als Verschlußschieber ausgebildet, der entlang des Mantels 22 verschiebbar ist, um so eine Ausgabeöffnung 24 freizugeben. Seitlich, etwa auf halber Höhe der Wirbelkammer 1 ist der Aufgabetrichter 3 angeordnet, in den die Einzelkomponenten, insbesondere Glasfasern und Bindemittel eindosiert werden. Der Aufgabetrichter 3 ist zur Wirbelkammer 1 hin mittels eines Schiebers 26 verschlossen, der zum Aufgeben des

Materials in die Wirbelkammer geöffnet werden kann. Im Bereich der untersten Stelle der Wirbelkammer 1 führte die Druckluftleitung 2 etwa tangential zum Zylindermantelumfang in die Wirbelkammer 1, wobei die einströmende Luft entgegen der Schüttrichtung des Materials aus dem Aufgabetrichter 3 einströmt. Die Ausgabeöffnung 24 wird durch eine Schütte begrenzt, die durch zueinander zulaufende Leitbleche 27 gebildet ist. Das durch Öffnen des Schiebers 23 aus der Wirbelkammer 1 durch die Schütte 27 herausfallende Material kann dann in der weiter oben beschriebenen Weise in Folienbeuteln abgefüllt oder kompaktiert, gegebenenfalls auch in anderer als vorstehend erläuterter Weise, und anschließend weiterverarbeitet werden.

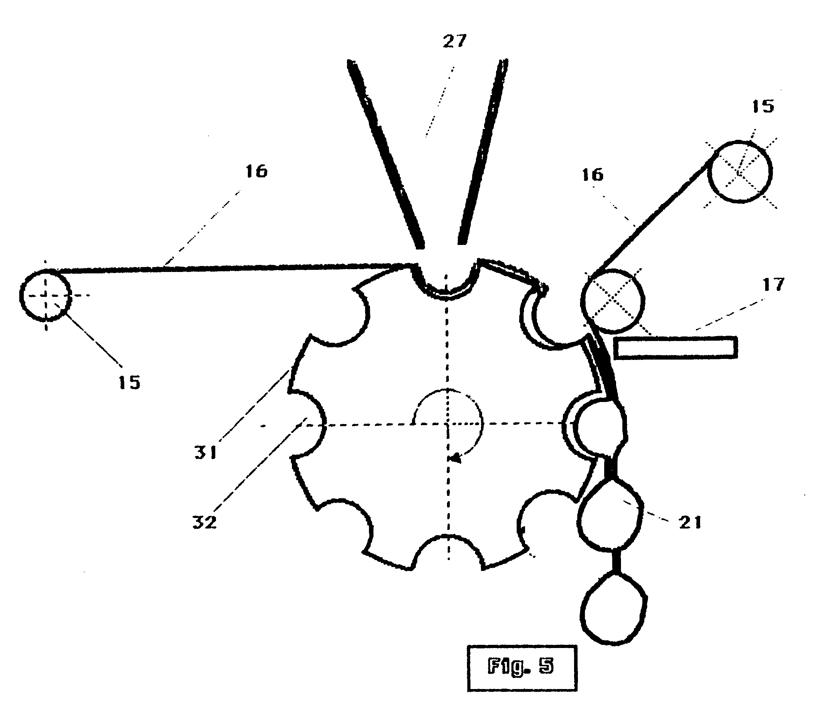

Insbesondere kann der Wirrfaserstoff auch in von der unter Bezugnahme auf die Figur 3 beschriebenen Weise abweichender Art verpackt werden. Dies ist in der Figur 5 dargestellt. Die Verpackungseinrichtung 14 der Figur 5 weist eine Zylinderwalze 31 auf, die auf ihrem Umfangsrand im Schnitt halbkreisförmige Ausnehmungen 32 hat, die stirnseitig begrenzt sind. Es sind weiterhin, wie bei der Ausgestaltung der Figur 3 zwei Zuführrollen 15 für je eine Folie 16 sowie eine Schweißeinrichtung 17 vorgesehen. Die Zylinderwalze 31 ist unter der Schütte 27 der Wirbelkammer 1 (Figur 4) angeordnet. Eine Folienbahn 16 wird in die Ausnehmung 32 im Mantel der Zylinderwalze 31 eingelegt. Anschließend wird die entsprechende Ausnehmung 32 mit eingelegter Folie 36 unter der Schütte 27 hindurchgeführt, wobei dies vorzugsweise im gleichen Takt geschieht wie die Verwirbelung einzelner Portionen in der Wirbelkammer 1 erfolgt. Eine in der Wirbelkammer 1 verwirbelte Portion des Wirrfaserstoffes rutscht in die entsprechende Ausnehmung 32. Beim nachfolgenden Weitertransport wird eine weitere Folie 16 über den in der Ausnehmung 32 befindlichen Wirrfaserstoff gelegt und an Mantelstellen der Zylinderwalze zwischen zwei Ausnehmungen 32 jeweils mittels der Schweißeinrichtung 17 mit der ersteingelegten Folie verschweißt, so daß einzelne Portionspackungen oder Beutel 21 gebildet werden.

**Patentansprüche**

1. Verfahren zur Herstellung eines Wirrfaserstoffs aus geschnittenen Faserbündeln und einem Bindemittel auf Polymerbasis als Ausgangsprodukt für die Herstellung faserverstärkter Kunststofformteile, wobei die Faserbündel auffilamentiert und verwirbelt und dabei mit dem Bindemittel versehen werden, dadurch gekennzeichnet, daß jeweils eine Charge aus aufeinander abgestimmten Mengen von Fasern und Bindemittel diskontinuierlich in eine Wirbelkammer (1) eingegeben, die Charge dort durch einen zugeführten turbulenten Druckluftstrom verwirbelt und anschließend als solche zur Weiterverarbeitung ausgegeben wird.

2. Verfahren nach Anspruch 1, dadurch gekennzeichnet, daß die Glasfaserstränge auffilamentiert werden bis Einzelfilamente vorliegen.

3. Verfahren nach Anspruch 1 oder 2, dadurch gekennzeichnet, daß die Glasfaserstränge von den beiden Enden aus auffilamentieren.

4. Verfahren nach einem der Ansprüche 1 bis 3, dadurch gekennzeichnet, daß Glasfasern und Bindemittel gleichzeitig der Verwirbelung zugeführt werden.

5. Verfahren nach einem der Ansprüche 1 bis 4, dadurch gekennzeichnet, daß das Bindemittel zusammen mit den Glasfasern in die Luftströmung eingegeben wird.

6. Verfahren nach einem der Ansprüche 1 bis 5, dadurch gekennzeichnet, daß die Glasfaserbündel und das Bindemittel in einen Druckluftstrom eingegeben und die mit Glasfasern beladene Luft in eine turbulente Wirbelströmung wechselnder Richtung umgelenkt wird.

7. Verfahren nach einem der Ansprüche 1 bis 6, dadurch gekennzeichnet, daß die Glasfaserbündel und das Bindemittel in der turbulenten Wirbelströmung großen Geschwindigkeitsänderungen ausgesetzt werden.

8. Verfahren nach einem der Ansprüche 1 bis 7, dadurch gekennzeichnet, daß pulverförmiges Bindemittel mit einer kantigen und rauhen Oberflächengestalt zugeführt wird.

9. Verfahren nach einem der Ansprüche 1 bis 8, dadurch gekennzeichnet, daß beim Verwirbeln die Komponenten elektrostatisch aufgeladen werden.

10. Verfahren nach einem der Ansprüche 1 bis 9, dadurch gekennzeichnet, daß der bindemittelhaltige Wirrfaserstoff nach seiner Bildung zu einem filzartigen Gebilde (9) verdichtet wird.

11. Vorrichtung zur Durchführung des Verfahrens nach Anspruch 1, mit einer Wirbelkammer (1), einer in diese mündenden Luftleitung (2) sowie Aufgabeeinrichtungen (3) für Faserbündel und Bindemittel, dadurch gekennzeichnet, daß die Wirbelkammer (1) luftdurchlässige Wände aus Filtermaterial und eine mit einem Schieber (4, 23) versehene Austrittsöffnung (24) für den Wirrfaserstoff aus Faser und Bindemittel aufweist.

12. Vorrichtung nach Anspruch 11, dadurch gekennzeichnet, daß die Druckluftleitung (2) ein in die Wirbelkammer (1) reichendes Mündungsstück (19) aufweist, das in sich ändernder Richtung mit Bezug auf die Wirbelkammer (1) beweglich ist.

13. Vorrichtung nach einem der Ansprüche 11 bis 12, dadurch gekennzeichnet, daß die Austrittsöffnung (24) sich über den Behälterquerschnitt erstreckt.

14. Vorrichtung nach einem der Ansprüche 11 bis 13, dadurch gekennzeichnet, daß die Aufgabeeinrichtung (3) in die Druckluftleitung (2) mündet.

15. Vorrichtung nach den Ansprüchen 13 und 14, dadurch gekennzeichnet, daß ein in die Wirbelkammer (1) von der der Austrittsöffnung gegenüberliegenden Seite eintauchender und bis zur Austragsöffnung beweglicher Austragsschieber (6) vorgesehen ist.

16. Vorrichtung nach einem der Ansprüche 11 bis 15, dadurch gekennzeichnet, daß die Aufgabeeinrichtung (3) mit Abstand zur Mündung der Druckluftleitung (2) in die Wirbelkammer (1) mündet.

17. Vorrichtung nach Anspruch 16, dadurch gekennzeichnet, daß die Wirbelkammer als liegender Zylinder (22, 23) ausgebildet ist, in dessen Mantel Druckluftleitung (2) und Aufgabeeinrichtung (3) münden und dessen Austrittsöffnung (24) im Mantel ausgebildet ist, und daß der Schieber (23) der Mantelkontur folgend verschwenkbar ausgebildet ist.

18. Vorrichtung nach Anspruch 17, dadurch gekennzeichnet, daß an dieser Austrittsöffnung (24) sich eine Schütte (27) anschließt.

19. Vorrichtung nach einem der Ansprüche 11 bis 18 zur Durchführung des Verfahrens nach Anspruch 6, dadurch gekennzeichnet, daß an die Austragsöffnung (24) eine Verdichtungseinrichtung (6, 12) angeschlossen ist.

20. Vorrichtung nach Anspruch 19, dadurch gekennzeichnet, daß die Verdichtungseinrichtung (6, 12) von dem Austragsschieber (6) und einer mit Abstand von der Austragsöffnung angeordneten Gegendruckplatte (12) gebildet ist.

21. Vorrichtung nach einem der Ansprüche 19 bis 20, dadurch gekennzeichnet, daß an die Verdichtungseinrichtung (6, 12) eine beheizte Bandpresse (10) zur Weiterverarbeitung des verdichteten Glasfaserfilzes (9) zu glasfaserverstärkten Kunststoffplatten als Zwischenprodukt für die Herstellung von Formteilen angeschlossen ist.

22. Vorrichtung nach einem der Ansprüche 11 bis 20, dadurch gekennzeichnet, daß hinter der Austrittsöffnung (6, 12) eine Einrichtung (14) zum Abpacken des Glasfaserfilzes (9) in Folienpackungen (mittels Folien 16) angeschlossen ist.

**Claims**

1. Process for the production cf a tangled fibrous material from cut fibre bundles and a polymer-based binder as the starting product for the production of fibre-reinforced plastic mouldings, in which the fibre bundles are filamented on and whirled and provided with the binder, characterized in that a charge of matched quantities of fibres and binder is discontinuously fed into vortex chamber (1), where the charge is whirled by a turbulent compressed air flow fed in and subsequently is discharged as such for further processing.

2. Process according to claim 1, characterized in that the glass fibre strands are filamented on until individual filaments are obtained.

3. Process according to claims 1 or 2, characterized in that the glass fibre strands are filamented on from both ends.

4. Process according to one of the claims 1 to 3, characterized in that the glass fibres and binders are simultaneously supplied for whirling.

5. Process according to one of the claims 1 to 4, characterized in that the binder is fed together with the glass fibres into the air flow.

6. Process according to one of the claims 1 to 5, characterized in that the glass fibre bundles and the binder are fed into a compressed air flow and the glass fibre-laden air is deflected into a turbulent vortex flow of alternating direction.

7. Process according to one of the claims 1 to 6, characterized in that the glass fibre bundles and binder are exposed to high speed changes in the turbulent vortex flow.

8. Process according to one of the claims 1 to 7, characterized in that pulverulent binder having an angular and rough surface form is supplied.

9. Process according to one of the claims 1 to 8, characterized in that the components are electrostatically charged during whirling.

10. Process according to one of the claims 1 to 9, characterized in that, following its formation, the binder-containing tangled fibrous material is compressed into a felt-like structure (9).

11. Apparatus for performing the process according to claim 1, with a vortex chamber (1), an air line (2) issuing into it, as well as feed mechanisms (3) for fibre bundles and binder, characterized in that the vortex chamber (1) has air-permeable walls made from filter material and an outlet (24), provided with a slide (4, 23), for the tangled fibrous material formed from fibres and binder.

12. Apparatus according to claim 11, characterized in that the compressed air line (2) has a mouthpiece (19) extending into the vortex chamber (1) and which is movable in varying directions with respect to the vortex chamber (1).

13. Apparatus according to one of the claims 11 or 12, characterized in that the outlet (24) extends over the container cross-section.

14. Apparatus according to one of the claims 11 to 13, characterized in that the feed mechanism (3) issues into the compressed air line (2).

15. Apparatus according to claims 13 and 14, characterized in that a discharge slide (6) is provided, which is introduced into the vortex chamber (1) from the side facing the discharge opening and which moves up to the latter.

16. Apparatus according to one of the claims 11 to 15, characterized in that the feed mechanism (3) issues into the vortex chamber (1) at a distance from the opening of the compressed air line (2).

17. Apparatus according to claim 16, characterized in that the vortex chamber is constructed as horizontal cylinders (22, 23), into whose casing issue the compressed air line (2) and feed mechanism (3) and whose outlet (24) is formed in the casing and that the slide (23) is constructed so as to pivotably follow the casing contour.

18. Apparatus according to claim 17, characterized in that a chute (27) is connected to said outlet (24).

19. Apparatus according to one of the claims 11 to 18 for performing the process according to claim 6, characterized in that a compression mechanism (6, 12) is connected to the discharge opening (24).

20. Apparatus according to claim 9, characterized in that the compression mechanism (6, 12) is formed by the discharge slide (6) and a counterpressure plate (12) positioned at a distance from the discharge opening.

21. Apparatus according to one of the claims 19 or 20, characterized in that to the compression mechanism (6, 12) is connected a heated belt press (10) for the further processing of the compressed glass fibre felt (9) to glass fibre-reinforced plastic plates as an intermediate for the production of mouldings.

22. Apparatus according to one of the claims 11 to 20, characterized in that behind the outlet (6, 12) is connected a device (14) for packing the glass fibre felt (9) in foil packs (by means of foils 16).

**Revendications**

1. Procédé pour fabriquer un tissu en fibres non orientées à partir de faisceaux de fibres coupées et d'un liant à base de polymère comme produit de départ, pour la fabrication de pièces moulées en matière plastique renforcées de fibres, les faisceaux de fibres étant défaits en filaments et mis en tourbillons, et ce faisant recevant le liant, caractérisé en ce qu'on introduit chaque fois dans une chambre de tourbillonnement (1), en discontinu, une charge constituée de quantités de fibres et de liant réglées l'une sur l'autre, qu'on y fait tourbillonner la charge à l'aide d'un courant d'air comprimé turbulent amené et qu'on l'envoie ensuite telle quelle à un traitement ultérieur.

2. Procédé selon la revendication 1, caractérisé en ce que les faisceaux de fibres de verre sont défaits en filaments jusqu'à obtention de filaments individuels.

3. Procédé selon la revendication 1 ou 2, caractérisé en ce que les faisceaux de fibres de verre sont défaits en filaments à partir des deux extrémités.

4. Procédé selon l'une des revendications 1 à 3, caractérisé en ce que les fibres de verre et le liant sont mis en tourbillons en même temps.

5. Procédé selon l'une des revendications 1 à 4, caractérisé en ce que le liant est introduit en même temps que les fibres de verre dans le courant d'air.

6. Procédé selon l'une des revendications 1 à 5, caractérisé en ce que les faisceaux de fibres de verre et le liant sont introduits dans un courant d'air comprimé et l'air chargé de fibres de verre est dévié dans un courant tourbillonnaire turbulent de sens alterné.

7. Procédé selon l'une des revendications 1 à 6, caractérisé en ce que les faisceaux de fibres de verre et le liant sont soumis dans le courant tourbillonnaire turbulent à de grandes variations de vitesse.

8. Procédé selon l'une des revendications 1 à 7, caractérisé en ce que le liant pulvérulent est amené avec une configuration de surface à arêtes vives et rugueuses.

9. Procédé selon l'une des revendications 1 à 8, caractérisé en ce que les constituants sont chargés électrostatiquement lors du tourbillonnement.

10. Procédé selon l'une des revendications 1 à 9, caractérisé en ce qu'après sa formation, le tissu de fibres non orientées contenant le liant est compacté pour former une structure (9) de type feutre.

11. Dispositif pour mettre en oeuvre le procédé selon la revendication 1, comportant une chambre de tourbillonnement (1), une canalisation d'air (2) débouchant dans celle-ci ainsi que des dispositifs d'alimentation (3) pour les faisceaux de fibres et le liant, caractérisé en ce que la chambre de tourbillonnement (1) comporte des parois en matériau filtrant laissant passer l'air et une ouverture de sortie (24) munie d'un tiroir (4,23) pour le tissu de fibres non orientées constitué de fibres et de liant.

12. Dispositif selon la revendication 11, caractérisé en ce que la canalisation d'air comprimé (2) comporte un embout (19) allant dans la chambre de tourbillonnement (1), qui peut être orienté dans une direction variable par rapport à la chambre de tourbillonnement (1).

13. Dispositif selon l'une des revendications 11 et 12, caractérisé en ce que l'ouverture de sortie (24) s'étend sur la section transversale du récipient.

14. Dispositif selon l'une des revendications 11 à 13, caractérisé en ce que le dispositif d'alimentation (3) débouche dans la canalisation d'air comprimé (2).

15. Dispositif selon les revendications 13 et 14, caractérisé en ce qu'on prévoit un tiroir de décharge (6) plongeant dans la chambre de tourbillonnement (1) à partir du côté opposé à l'ouverture de sortie et pouvant se déplacer jusqu'à l'ouverture de décharge.

16. Dispositif selon l'une des revendications 11 à 15, caractérisé en ce que le dispositif d'alimentation (3) débouche dans la chambre de tourbillonnement (1) à une certaine distance de l'embouchure de la canalisation d'air comprimé (2).

17. Dispositif selon la revendication 16, caractérisé en ce que la chambre de tourbillonnement est réalisée sous forme d'un cylindre couché (22,23) dans l'enveloppe duquel débouchent la canalisation d'air comprimé (2) et le dispositif d'alimentation (3) et dont l'ouverture de sortie (24) est formée dans l'enveloppe, et en ce que le tiroir (23) est réalisé de façon à pouvoir pivoter en suivant le contour de l'enveloppe.

**18.** Dispositif selon la revendication 17, caractérisé en ce qu'une gouttière (27) est raccordée à cette ouverture de sortie (24).

**19.** Dispositif selon l'une des revendications 11 à 18, pour mettre en oeuvre le procédé selon la revendication 6, caractérisé en ce qu'un dispositif de compactage (6,12) est raccordé à cette ouverture de décharge (24).

**20.** Dispositif selon la revendication 19, caractérisé en ce que le dispositif de compactage (6,12) est constitué par le tiroir de décharge (6) et une plaque de contre-pression (12) disposée à une certaine distance de l'ouverture de décharge.

**21.** Dispositif selon l'une des revendications 19 et 20, caractérisé en ce qu'on raccorde au dispositif de compactage (6,12) une presse à bande chauffée (10) pour appliquer au feutre de fibres de verre (9) un traitement ultérieur pour obtenir des plaques de matière plastique renforcées de fibres de verre en tant que produit intermédiaire pour la fabrication de pièces moulées.

**22.** Dispositif selon l'une des revendications 11 à 20, caractérisé en ce qu'en aval de l'ouverture de sortie (6,12) on raccorde un dispositif (14) pour emballer le feutre de fibres de verre (9) en emballages sous film (au moyen des films 16).

Bereitstellen

Glasfaser

Wiegen, Dosieren, Fördern

Luft

Mischen
Ausstoßen

Halbzeugherstellung

Folie

16

21

Lagern, Wiegen, Dosieren,
Fördern : Bindemittel

Bunker

Fördern

Filter

1

3

4

9

Bauteilherstellung

Komprimierstation

Weiterverarbeitung

Fördern, Komprimieren,
Einzelverpackung,
Vakuum ziehen,
Lagern

Fig. 1

Fig. 2

Fig. 3

13

Eindosieren der
Einzelkomponenten

26

Schieber

22          5

1                           Filter

3

Luft- und
Mischbewegung

Luft

2                       24

27                                    23

27

Zur Weiterverarbeitung

Fig. 4

14

Fig. 5